# FASCICULE DE BREVET EUROPEEN

(11) **EP 2 694 516 B1**
(45) Date de publication et mention de la délivrance du brevet: **17.06.2015**
(21) Numéro de dépôt: 12720240.6
(22) Date de dépôt: 05.04.2012
(51) Int. Cl.: C07D 487/22, C30B 29/54, C30B 29/60, C06B 25/34

(54) **PROCEDE D'OBTENTION D'UNE CHARGE DE CRISTAUX D'HEXANITROHEXAAZAISOWURTZITANE A MORPHOLOGIE ARRONDIE; CHARGE ET MATERIAU ENERGETIQUE CORRESPONDANT.**
VERFAHREN ZUR HERSTELLUNG VON EINER HEXANITROHEXAAZAISOWURTZITANE KRISTALL-CHARGE MIT ABGERUNDETEN MORPHOLOGIE; CHARGE UND ENERGETISCHE MATERIAL DAVON
METHOD FOR OBTAINING A BATCH OF HEXANITROHEXAAZAISOWURTZITANE CRYSTALS HAVING A ROUND MORPHOLOGY; BATCH AND ENERGETIC MATERIAL THEREOF

(30) Priorité: 08.04.2011 FR 1153065
(43) Date de publication de la demande: 12.02.2014
(73) Titulaire: HERAKLES, 33185 Le Haillan (FR); Eurenco, 91300 Massy (FR)
(72) Inventeur: MUSCATELLI, Florent, F-91710 Vert Le Petit (FR); LESCOP, Philippe, F-91710 Vert Le Petit (FR)
(74) Mandataire: Le Roux, Martine
(86) Numéro de dépôt international: PCT/FR2012/050733
(87) Numéro de publication internationale: WO 2012/136933

(56) Documents cités:
- EP-A1- 0 913 374
- DE-A1- 3 605 634
- FR-A1- 2 858 620
- FR-A1- 2 887 544
- FR-A1- 2 950 623
- US-A1- 2007 225 493

## Description

La présente invention a pour objet un procédé pour l'obtention d'une charge de cristaux d'hexanitrohexaazaisowurtzitane, lesdits cristaux présentant une morphologie arrondie. Elle concerne également ladite charge *per se,* le matériau énergétique la renfermant et un procédé de fabrication dudit matériau.

L'invention se situe dans le domaine des poudres, propergols et explosifs, utilisés notamment dans les industries d'armement.

Les cristaux de l'invention, obtenus sous la forme de charges (de cristaux non maclés), présentent une forme cristallisée arrondie particulièrement adaptée à la formulation de matériaux énergétiques. Ceci est ci-après explicité en détail.

Il existe, depuis quelques années, de nombreuses publications relatives au 2,4,6,8,10,12-hexanitro-2,4,6,8,10,12-hexaazatétracyclo (5.5.0.0^{5,9}.0^{3,11}) dodécane, encore appelé hexanitrohexaazaisowurtzitane ou CL20. Ces publications décrivent les diverses formes polymorphes de ce composé (il est en effet connu que l'hexanitrohexaazaisowurtzitane peut être obtenu sous quatre formes polymorphes cristallines : beta, alpha, gamma et epsilon), les propriétés physiques, chimiques et détoniques de ce composé ainsi que l'utilisation dudit composé dans des compositions explosives, des propergols ou des poudres pour armes.

C'est la forme polymorphe epsilon dudit composé (CL20 ε) qui possède la masse volumique la plus élevée (2,04 g/cm³) et qui présente donc le plus d'intérêt, notamment pour utilisation dans les compositions pyrotechniques.

La demande de brevet EP-A-0 913 374 décrit un procédé d'obtention du CL20 ε selon les étapes réactionnelles suivantes :
- on réalise tout d'abord une solution saturée de CL20 de forme polymorphe quelconque, de préférence autre que la forme epsilon, dans un mélange comprenant, d'une part, un solvant organique du CL20 choisi dans le groupe constitué par les esters, les nitriles, les éthers, les cétones à l'exclusion de l'acétone et leurs mélanges, et, d'autre part, un non solvant organique du CL20 choisi dans le groupe constitué par les hydrocarbures aliphatiques, les hydrocarbures aromatiques et leurs mélanges, ledit solvant du CL20 étant plus volatil que ledit non solvant (i.e. présentant une pression de vapeur saturante inférieure à celle du non solvant) (ledit solvant et ledit non solvant étant évidemment miscibles dans les proportions utilisées pour la constitution dudit mélange renfermant le CL20 (pour l'obtention d'une solution saturée de CL20)),
- on ensemence ensuite cette solution saturée par quelques cristaux de CL20 ε, puis
- on concentre ladite solution saturée ensemencée par évaporation du solvant (ce qui provoque l'apparition de cristaux de CL20 ε dans le mélange enrichi en non solvant).

La solution saturée de CL20 peut être préparée selon deux modes opératoires : préparation d'un mélange solvant + non solvant, puis ajout audit mélange de CL20 à saturation ou préparation d'un mélange non solvant + CL20 en excès, puis ajout audit mélange du solvant, puis filtration du CL20 en excès.

La charge de cristaux obtenue(les cristaux obtenus) peu(ven)t être récupérée(récupérés), par toute méthode usuelle, telle que la filtration.

Comme exemples de solvant organique du CL20, on peut citer le formiate de méthyle, l'acétate de méthyle, l'acétate d'éthyle, l'acétate d'isopropyle, l'acétonitrile, les mélanges acétate d'éthyle-acétonitrile, le tétrahydrofurane (THF) et la méthyléthylcétone. L'acétone, exclue de la liste des solvants utilisables, l'a été dans la mesure où la mise en oeuvre du procédé avec ce solvant (plus précisément avec le couple solvant/ non solvant = acétone/ toluène) conduisait à des cristaux agglomérés, les agglomérats ou macles présentant un diamètre supérieur à 100 µm (voir l'exemple 4 de la demande EP-A-0 913 374).

Comme exemples de non solvants organiques du CL20, on peut citer le toluène, les xylènes, les alcanes tels que l'hexane, l'heptane et l'octane ainsi que les hydrocarbures aliphatiques halogénés, notamment les hydrocarbures aliphatiques chlorés tels que le 1,2-dichloroéthane,

Une autre famille de non solvant organique du CL20, dont l'utilisation dans le procédé ci-dessus (selon la demande EP-A-0 913 374) est décrite dans la demande de brevet FR-A-2 950 623, consiste en les hydrofluoroéthers ininflammables, tel que le 2-trifluorométhyl-3-éthoxy-1,1,1,2,3,4,4,5,5,6,6,6-dodécafluorohexane (dénommé plus simplement ci-après le 2-trifluorométhyl-3-éthoxydodécafluorohexane), de formule brute (CF₃)C₆F₁₂OC₂H₅. Ce composé chimique porte le numéro CAS 297730-93-9. Il est notamment commercialisé par la société 3M sous la dénomination commerciale HFE 7500.

Pour la mise en oeuvre du procédé ci-dessus, les couples solvant/non solvant : acétate d'éthyle/toluène et acétate d'éthyle/HFE7500 sont particulièrement préférés.

Le rapport massique solvant/non solvant est généralement compris entre 10/90 et 50/50, avantageusement entre 15/85 et 35/65.

Lors de la concentration de la solution (mélange solvant/non solvant saturé en CL20) par évaporation du solvant organique, la température n'excède pas 50°C à 70°C selon la nature de la solution en cause et ce, en référence à la pureté des cristaux de CL20 recherchés.

La demande de brevet EP-A-0 913 374 décrit, dans son exemple 5, pour un couple solvant/non solvant constitué d'acétate d'éthyle/toluène, une évaporation du solvant mise en oeuvre à la température ambiante (proche de 20°C) et à une faible pression de 6,25 10³ Pa (62,5 mbar, environ 50 mmHg). Une telle pression est très inférieure à la pression d'ébullition du solvant dans la solution.

A l'échelle industrielle, on préfère travailler à température plus élevée (environ 50°C) et à une pression moins faible (∼ 25 10³ Pa, soit 250 mbar), qui demeure toutefois très inférieure à la température d'ébullition du solvant dans la solution. De telles conditions opératoires sont intéressantes sur le plan de la mise en oeuvre du procédé, car il est aisé de procéder à température ambiante ou à chaud et avec une faible pression de soutirage du solvant, conduisant à une évaporation rapide dudit solvant, ce qui permet de réduire la durée de mise en oeuvre du procédé. Cependant, l'application d'une faible pression, très inférieure à la pression d'ébullition du solvant dans la solution, dès le début du processus d'évaporation, provoque une ébullition intense dudit solvant (un effet moussant se développe alors), difficilement compatible avec une maîtrise reproductible du procédé, notamment en début de soutirage dudit solvant.

Les cristaux de CL20 brut obtenus se présentent sous la forme de bipyramide, le plus souvent maclés. Ces cristaux présentent un rapport d'allongement (voir la définition ci-après de ce paramètre) supérieur à 2, voire supérieur à 4, pour plus de 90% (en nombre), voire pour la totalité, d'entre eux.

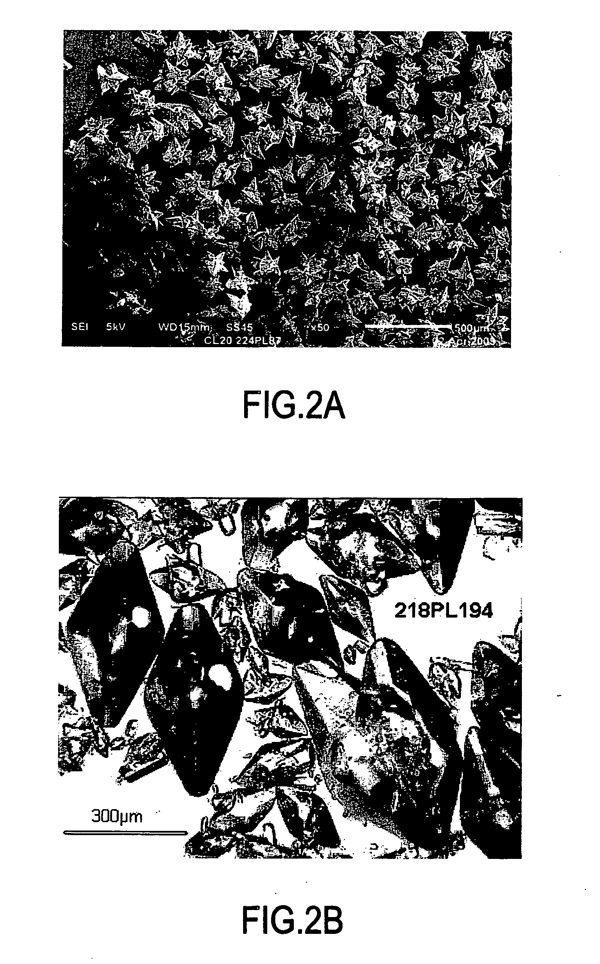

De tels cristaux sont du même type que ceux montrés sur la figure 2A (cristaux maclés obtenus avec le couple solvant/ non solvant : acétate d'éthyle/ HFE 7500) et sur la figure 2B (cristaux obtenus avec le couple solvant/ non solvant : acétone/toluène, montrés à plus fort grossissement) annexées (photographies prises au microscope optique à transmission). Ces figures sont à considérer avec les exemples ci-après.

Une telle morphologie de ses cristaux rend le CL20 peu adapté à la formulation dès que l'on vise des taux de charge importants. En effet, l'obtention de compositions à fort taux de charge est compromise du fait de la forte augmentation de viscosité inéluctablement associée à des taux de charge importants. Par ailleurs, la présence d'arêtes vives sur les cristaux leur confère une sensibilité accrue aux épreuves standards de sécurité pyrotechnique.

L'homme du métier souhaite donc disposer de cristaux de CL20 de forme arrondie (arrondie = compact, sans aiguille, par exemple cubique ou quasi sphérique) obtenus par un procédé simple et maîtrisé.

La demande de brevet CN-A-101624439 propose, à cette fin, l'obtention du CL20 par un procédé du même type que celui déjà décrit par la Demanderesse dans la demande de brevet FR-A-2 884 244 pour l'obtention de cristaux de dinitroamidure d'ammonium. Ce procédé consiste à introduire dans une solution contenant le CL20 une quantité efficace et non excessive d'au moins un modificateur de faciès cristallin, ledit au moins un modificateur de faciès cristallin pilotant, lors de la génération des cristaux, la croissance des faces cristallines et conduisant à l'obtention de cristaux de CL20 arrondis. D'une part, ce procédé n'est pas transposable à l'échelle industrielle sans une nécessaire étape préalable de qualification industrielle, et d'autre part, il nécessite l'adjonction de modificateur(s) de faciès dont il reste inéluctablement des traces polluant le produit final.

Dans un tel contexte, en référence au problème technique de la formulation des cristaux de CL20 (formulation à de forts taux de charge), la Demanderesse propose une solution tout à fait originale, surmontant les préjugés de l'homme du métier faisant état de l'impossibilité d'obtenir par simple cristallisation des cristaux de CL20 arrondis, mieux adaptés à la formulation de matériaux énergétiques. Cette solution repose ni sur un conditionnement des cristaux bruts (par érosion mécanique par exemple), ni sur l'intervention d'un modificateur du faciès cristallin lors de la génération des cristaux mais sur une maîtrise des paramètres de la croissance cristalline (des paramètres du procédé de cristallisation), permettant l'obtention de cristaux arrondis à gamme granulométrique choisie, pouvant aller de quelques microns à plusieurs centaines de microns.

Modifier les paramètres de croissance cristalline (les paramètres du procédé de cristallisation) pour générer des cristaux sous une forme cristallisée originale n'est pas une opération innovante en elle-même. Les paramètres en cause, tels la nature du solvant, notamment sa viscosité pour piloter les vitesses relatives de transfert et d'intégration des atomes au cristal, les cycles de température pour déplacer l'équilibre de la solution sur le diagramme de solubilité, la présence d'impuretés, l'agitation, sont connus de l'homme du métier. La modification desdits paramètres n'a toutefois jamais été décrite, encore moins maîtrisée, à la connaissance de la Demanderesse, dans le contexte de l'obtention de cristaux de CL20, en référence au problème technique de la formulation d'objets pyrotechniques.

Dans ce contexte particulier, les inventeurs ont montré, de façon tout à fait surprenante, qu'il est possible de mettre en oeuvre le procédé de cristallisation du CL20 en solution dans un mélange solvant/non solvant (procédé du type décrit dans les demandes EP-A-0 913 374 et FR-A-2 950 623) en vue d'obtenir des cristaux arrondis, tout en conservant le mode opératoire classique mais en imposant des paramètres d'évaporation du solvant (faible température et pression ajustée et contrôlée).

Selon son premier objet, la présente invention concerne donc un procédé d'obtention de cristaux de CL20. Il s'agit d'un procédé du type de celui décrit dans la demande EP-A-0 913 374. Ledit procédé constitue en fait un perfectionnement audit procédé selon la demande EP-A-0 913 374. Ce perfectionnement est particulièrement intéressant en référence à l'exploitation, à l'échelle industrielle, dudit procédé selon la demande EP-A-0 913 374.

Le procédé de l'invention est plus précisément un procédé d'obtention d'une charge de cristaux d'hexanitrohexaazaisowurtzitane, qui comprend :
- la préparation d'une solution saturée en hexanitrohexaazaisowurtzitane de forme polymorphe quelconque dans un mélange comprenant, d'une part, un solvant organique dudit hexanitrohexaazaisowurtzitane et, d'autre part, un non solvant organique dudit hexanitrohexaazaisowurtzitane ; ledit solvant étant plus volatil que ledit non solvant, (lesdits solvant et non solvant étant évidemment, dans les proportions où ils interviennent, miscibles, pour la constitution du mélange renfermant le CL 20 (i.e. pour la constitution de ladite solution saturée),
- l'ensemencement de cette solution saturée par quelques cristaux d'hexanitrohexaazaisowurtzitane, puis
- la concentration de la solution saturée ensemencée, par évaporation au moins partielle du solvant.

En cela, le procédé de l'invention est un procédé du type de celui décrit dans les demandes EP-A-0 913 374 et FR-A-2 950 623.

De façon caractéristique, selon l'invention, l'évaporation du solvant (pour la concentration de la solution saturée ensemencée) est mise en oeuvre à une température comprise entre 35°C et 15°C et à une pression ajustée, à plus ou moins 1,2 10³ Pa (+/-12 mbar), à la pression d'ébullition du solvant dans ladite solution, tout au long du processus d'évaporation dudit solvant de ladite solution.

Ledit solvant organique du CL20 est avantageusement choisi dans le groupe constitué par les esters, les nitriles, les éthers, les cétones et leurs mélanges et/ou, avantageusement et, ledit non solvant organique du CL20 est avantageusement choisi dans le groupe constitué par les hydrocarbures aliphatiques (tels les alcanes et notamment l'hexane, l'heptane et l'octane), les hydrocarbures aliphatiques halogénés (tels les hydrocarbures aliphatiques chlorés et notamment le 1,2-dichloroéthane), les hydrocarbures aromatiques (tel que le toluène et les xylènes), les hydrofluoroéthers ininflammables (tel que le 2-trifluorométhyl-3-éthoxydodécafluorohexane), et leurs mélanges. L'acétone (solvant) n'est présentement pas exclue dans la mesure où le procédé de l'invention, de par son optimisation des paramètres température et pression, en rend possible l'utilisation (notamment dans le cadre de l'utilisation d'un couple solvant/non solvant = acétone/toluène) pour l'obtention de charges de cristaux intéressantes.

On comprend que la pression appliquée, proche, à plus ou moins 1,2 10³ Pa (+/-12 mbar) près, de celle d'ébullition du solvant dans la solution, décroît ainsi au fur et à mesure du soutirage du solvant par évaporation (la concentration en solvant dans la solution diminuant au fur et à mesure de son soutirage, sa pression d'ébullition à température constante diminuant en conséquence).

La pression (d'évaporation) appliquée pendant le soutirage du solvant de la solution (le soutirage au mélange solvant/non solvant chargé en CL20) est avantageusement égale à plus ou moins 5 10² Pa (5 mbar) à la pression d'ébullition dudit solvant, très avantageusement égale à plus ou moins 1 mbar à la pression d'ébullition dudit solvant.

Le procédé de l'invention est donc mis en oeuvre à une température constante, pas très élevée, et à une pression non constante, évolutive, coïncidant, à plus ou moins 1,2 10³ Pa (+/- 12 mbar) près, à la pression d'ébullition du solvant dans le mélange solvant/non solvant (pression du solvant évolutive du fait de l'évaporation progressive dudit solvant). On comprend qu'un étalonnage préalable, pour chaque couple solvant/ non solvant, doit être mis en oeuvre.

Avantageusement, au démarrage de l'évaporation (du soutirage) du solvant, la pression appliquée à la solution (mélange solvant/non solvant) est supérieure à la pression d'ébullition du solvant dans les limites indiquées ci-dessus. La proportion de solvant dans la solution étant élevée audit démarrage de l'évaporation, on évite ainsi une ébullition intense dudit solvant (et donc une agitation de la solution par ébullition « moussante »), particulièrement marquée à basse température et pouvant induire des effets non maîtrisés sur le processus de cristallisation.

La température d'évaporation est comprise entre 35°C et 15°C.

Le contrôle des paramètres température (paramètre fixe) et pression (évolutive) d'évaporation du solvant s'est révélé strictement indispensable pour conduire à la production de cristaux de CL20 arrondis.

Les teneurs en solvant et cristaux (en suspension) de la suspension obtenue à l'issue de la mise en oeuvre de la concentration dépendent bien évidemment du mode exact de mise en oeuvre de cette concentration (du taux d'évaporation du solvant mis en oeuvre). L'évaporation du solvant peut être plus ou moins conséquente. Les cristaux peuvent ainsi être obtenus en suspension dans un mélange solvant/non solvant dont la phase liquide renferme notamment moins de 15 % en masse de solvant, avantageusement moins de 5% en masse dudit solvant. Si l'on vise à obtenir des suspensions (de cristaux dans le non solvant) quasi exemptes de solvant, le procédé de l'invention comprend l'évaporation dudit solvant de la solution ensemencée (évaporation avantageusement conséquente) et en outre le lavage, avec le non solvant, de la suspension obtenue.

La suspension obtenue, ayant pour phase liquide le non solvant ou un mélange solvant/non solvant (avec un taux plus ou moins conséquent de solvant), peut, selon une première variante, être conservée en l'état et utilisée dans un procédé de fabrication d'un matériau énergétique incluant l'extraction (totale ou quasi totale) de ladite phase liquide (voir plus loin). Ladite suspension peut, selon une autre variante, être filtrée afin de récupérer les cristaux (la charge de cristaux ou phase solide) qu'elle contient ; lesdits cristaux étant ensuite utilisés dans un procédé de fabrication d'un matériau énergétique. L'une ou l'autre de ces variantes est opportunément mise en oeuvre au vu de la nature exacte de la phase liquide (notamment de la nature du non solvant) de la suspension.

Avantageusement, l'ensemencement de la solution saturée est réalisé par des cristaux d'hexanitrohexaazaisowurtzitane de forme polymorphe epsilon (ε), afin d'obtenir une suspension de cristaux d'hexanitrohexaazaisowurtzitane de forme polymorphe epsilon (ε) dans une phase liquide. Selon cette variante de mise en oeuvre, ladite solution saturée est de préférence saturée en CL20 d'une forme polymorphe autre que la forme epsilon (ε).

Les couples solvant/non solvant préférés sont les suivants : acétate d'éthyle/HFE 7500, acétate d'éthyle/toluène et acétone/toluène. Le couple acétate d'éthyle/HFE 7500 est tout particulièrement préféré.

Le ratio en masse du couple solvant/non solvant est typiquement compris entre 10/90 et 50/50, avantageusement entre 15/85 et 35/65. Il est généralement ajusté pour une solubilité à saturation du CL20 de 100 g/L à 200 g/L, afin, d'une part, de limiter les risques pyrotechniques et, d'autre part, d'éviter une possible abrasion des cristaux par frottement entre eux dans la solution.

Le procédé de l'invention, de par la maîtrise des conditions d'évaporation du solvant, conduit à l'obtention de cristaux (et non d'agglomérats ou macles de cristaux), de forme arrondie particulièrement intéressante, différents de ceux de l'art antérieur sous la forme de bipyramide, le plus souvent maclés. Il ne conduit pas à l'obtention de seulement quelques cristaux de ce type. Le procédé de l'invention conduit plus précisément à l'obtention d'une charge de cristaux de ce type. Ladite charge renferme ainsi avantageusement au moins 80 % (en nombre), très avantageusement plus de 80 % (en nombre), voire plus de 90 % (en nombre), de cristaux (cristaux non maclés), présentant un rapport d'allongement inférieur ou égal à 1,5. Ledit rapport d'allongement est très généralement compris entre 1,5 et 1. On note par ailleurs que la charge des cristaux de l'invention n'est pas polluée (que les cristaux constituant ladite charge ne sont pas pollués) par l'intervention d'un quelconque modificateur de facies cristallin (lors de la croissance desdits cristaux).

Le paramètre "rapport d'allongement", utilisé en référence aux cristaux de l'invention, est classiquement défini comme le rapport, pour un cristal, de son diamètre de Féret maximum sur son diamètre de Féret minimum ; ledit diamètre de Féret maximum correspondant à la distance maximale entre deux tangentes parallèles sur des côtés opposés du cristal et ledit diamètre de Féret minimum correspondant à la distance minimale entre deux tangentes parallèles sur des côtés opposés du cristal.

Il est aussi remarquable que lesdits cristaux présentent par rapport à ceux l'art antérieur une sensibilité réduite, comme montré dans les exemples ci-après.

Selon son second objet, la présente invention concerne donc une charge de cristaux susceptible d'être obtenue par le procédé décrit ci-dessus (premier objet de l'invention) et plus particulièrement une charge présentant les caractéristiques énoncées ci-dessus. On a compris, au vu des propos ci-dessus, qu'une telle charge de cristaux peut exister sous la forme d'une suspension (cristaux en suspension dans une phase liquide) ou « à l'état sec » (cristaux récupérés après filtration).

Selon son troisième objet, la présente invention concerne un matériau énergétique renfermant au moins une telle charge, à l'état sec (charge *per se* ou telle qu'obtenue par ledit procédé). Ledit matériau énergétique renferme donc des cristaux d'hexanitrohexaazaisowurtzitane et au moins une partie desdits cristaux consiste en des cristaux d'une charge de l'invention. Lesdits cristaux peuvent être présents dispersés dans un liant, énergétique ou non. La présence d'un liant n'est toutefois nullement obligatoire. Le matériau énergétique renferme au moins une charge de cristaux à l'état sec (charge de l'invention) mais est susceptible d'avoir été préparé à partir d'une telle charge de cristaux à l'état sec ou à partir d'une suspension de cristaux (voir ci-après). Sa fabrication selon cette seconde variante constitue le quatrième et dernier objet de l'invention.

Selon son quatrième objet, la présente invention concerne donc la fabrication d'un matériau énergétique renfermant des cristaux d'hexanitrohexaazaisowurtzitane. Le procédé de fabrication, selon l'invention, dudit matériau comprend, de façon classique, l'introduction et le malaxage des ingrédients (liant, agent de réticulation, charge(s)...) dans un malaxeur (pour la constitution d'une pâte). De façon caractéristique, il comprend l'introduction de cristaux de CL20 non pas sous la forme de cristaux mais au sein d'une suspension obtenue selon l'invention (charge de cristaux selon l'invention sous la forme d'une suspension (dont la phase liquide est un mélange solvant/non solvant, avantageusement riche en non solvant, voire quasi exclusivement constitué du non solvant)). Ladite suspension est utilisée comme source desdits cristaux, comme ingrédient ou matière première. En son sein, les cristaux sont flegmatisés. La phase liquide de ladite suspension est ensuite évacuée par extraction, extraction sous vide, totale ou quasi-totale. Ce procédé de fabrication, qui utilise donc une suspension de cristaux comme source desdits cristaux, peut être mis en oeuvre à l'issue de l'obtention de ladite suspension ou en différé par rapport à ladite obtention (après conservation de la suspension obtenue, ou après transport de celle-ci, ou après conservation et transport de celle-ci, ou encore après transport et conservation de celle-ci). On comprend que le matériau énergétique peut ainsi être obtenu avec une grande souplesse. La flegmatisation des cristaux au sein de la suspension, d'où découle cette souplesse de mise en oeuvre du procédé, est particulièrement avantageuse.

Notons qu'il est tout à fait possible, dans le cadre de la mise en oeuvre d'un autre procédé de fabrication d'un matériau énergétique, d'utiliser des cristaux secs issus du procédé de la présente invention (au moins une charge de cristaux à l'état sec), ou des cristaux séchés de la présente invention (au moins une charge de cristaux à l'état sec) et mis en suspension dans un autre non solvant (eau par exemple).

L'invention est maintenant illustrée, de façon nullement limitative, par les figures annexées et exemples ci-après.

Le HFE 7500 (2-trifluorométhyl-3-éthoxydodécafluorohexane) utilisé est commercialisé par la société 3 M.

Les figures 1A et 1B montrent des cristaux arrondis obtenus selon le procédé de l'invention, mis en oeuvre respectivement avec une solution solvant/non solvant : acétate d'éthyle/HFE (1A) et acétone/toluène (1B) (voir les exemples ci-après).

Les figure 2A et 2B montrent des cristaux en forme de bypiramide maclés, obtenus respectivement avec une solution solvant/non solvant : acétate d'éthyle/HFE 7500 (2A) et acétone/toluène (2B), dans des conditions (de température) qui ne sont pas celles de l'invention (voir les exemples comparatifs ci-après).

### Exemple A et exemple comparatif B

Le procédé de l'invention est mis en oeuvre, conformément à l'exemple A du tableau 3 (voir ci-après), dans un réacteur d'un volume de 60 L. La cristallisation par évaporation est mise en oeuvre à partir d'une solution : acétate d'éthyle (solvant)/HFE 7500 (non solvant), saturée en CL20, lesdits solvant, non solvant et CL20 étant présents respectivement dans les proportions massiques suivantes : 30,1 %/61,8 %/8,1 %.

On trouve comme élément habituel sur le montage de distillation, un mobile d'agitation qui possède un profil mince, peu brisant, avec cependant une bonne capacité de pompage, permettant de pallier à la décantation des cristaux dans des régimes de rotation tout en évitant la brisure en bout de pale. Ceci permet, lors de l'opération, de continuellement faire passer les cristaux en phase de croissance à l'interface d'évaporation (lieu où la solution se désature) sans trop les solliciter "mécaniquement".

Après introduction, dans le réacteur, de la solution de départ saturée en CL20, on porte la température de ladite solution à 20°C puis on introduit la semence (110 g de CL20 ε de taille moyenne 30 µm). Dans ces conditions, la pression d'ébullition du solvant de la solution (solvant/non solvant) est de 17 10³ Pa (170 mbar). Le processus d'évaporation débute en appliquant à la solution une pression de soutirage de 18 10³ Pa (180 mbar), légèrement supérieure (+ 10 mbar) à la pression d'ébullition du solvant, afin d'éviter une ébullition massive du solvant susceptible de perturber (par agitation de la solution) le phénomène de cristallisation du CL20. On soutire alors progressivement pendant 5h30 l'acétate d'éthyle par ajustement de la pression appliquée à la solution dans les limites de +/-1 10³ Pa (+/- 10 mbar) autour de la pression d'ébullition dudit solvant. La pression appliquée suit donc une valeur consignée à l'intérieur du gabarit imposé. La pression en fin de soutirage est de 80 mbar. La proportion massique en solvant en fin de soutirage est d'environ 10 %. On revient alors à la pression atmosphérique et on vidange la suspension de CL20 ε dans le mélange solvant/non solvant.

Pour analyser, par microscopie électronique, les cristaux de CL20 ε de la suspension, la suspension est filtrée et le rétentat essoré. Le produit est ensuite séché en étuve.

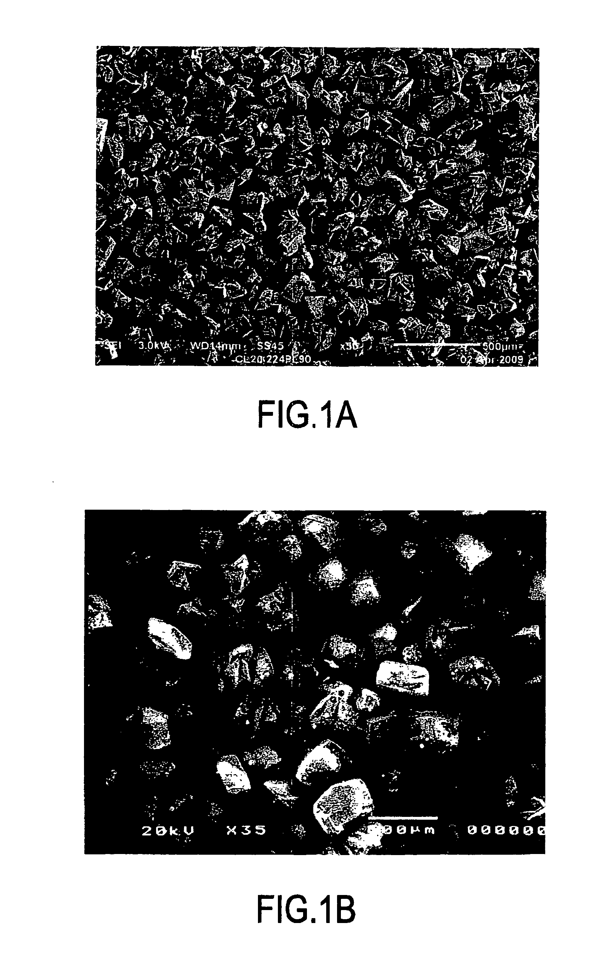

On récupère sur le filtre un produit cristallin, de couleur "blanc cassé", composé de cristaux arrondis de CL20 ε d'un diamètre médian de 122 µm, non maclés, de rapport d'allongement inférieur à 1,5 pour plus de 90 % d'entre eux (% en nombre), tels que montrés sur la figure 1A.

L'exemple B du tableau 3 concerne l'obtention de cristaux à chaud tout en contrôlant le paramètre pression comme selon l'invention. Pour une solution saturée proche de celle de l'exemple A (voir ledit tableau 1), on distille progressivement le solvant pendant 2h10 en portant la température de distillation à 67°C et en ajustant la pression appliquée à la solution légèrement en dessous du point d'ébullition du solvant, la pression variant alors entre 42,5 10³ Pa (425 mbar) et 32,5 10³ Pa (325 mbar). En fin de distillation, la proportion massique en solvant en fin de soutirage est d'environ 5%. On récupère sur le filtre un produit cristallin, de couleur "blanc cassé", composé de cristaux de CL20 ε, sous la forme de bipyramide, de rapport d'allongement supérieur à 2 pour plus de 90% d'entre eux (% en nombre), le plus souvent maclés, d'un diamètre médian de 115 µm, tels que montrés sur la figures 2A.

Cet exemple comparatif B démontre que l'ajustement du paramètre pression (selon l'invention) n'est pas suffisant pour obtenir les cristaux de l'invention. Il est nécessaire de combiner selon l'invention une température relativement basse et un ajustement de la pression pour obtenir la morphologie cristalline désirée.

Les cristaux obtenus selon le procédé de l'invention présentent donc une forme arrondie favorable à l'obtention de matériaux énergétiques les incorporant à fort taux de charge.

De plus, ils présentent une sensibilité réduite aux tests de sensibilité pyrotechnique, par exemple au test de sensibilité à l'impact (ISI)*, et au test de sensibilité du type "amorçage de la détonation à travers une barrière" aussi appelé IAD* (Indice d'Aptitude à la Détonation) : voir les résultats indiqués dans le tableau 1 ci-après. Des précisions sur lesdits tests sont données plus loin.

**Tableau 1**

| | Propriétés des cristaux obtenus | | |
|---|---|---|---|
| | Dmédian (µm) | ISI (J) | IAD (cartes) |
| ***A (invention)*** | 122 | 4 | 335 |
| ***B (exemple comparatif)*** | 115 | 2,7 | > 360 |

### Exemple C et exemple comparatif D :

Le procédé de l'invention est mis en oeuvre, conformément à l'exemple C du tableau 4 (voir ci-après), dans un réacteur d'un volume de 60 L. La cristallisation par évaporation est mise en oeuvre à partir d'une solution : acétone (solvant)/toluène (non solvant), saturée en CL20, lesdits solvant, non solvant et CL20 étant présents respectivement dans les proportions massiques suivantes 18,1 %/72,6 %/9,3 %.

On distille progressivement le solvant pendant 5h00 en portant la température de distillation à 20°C et en ajustant la pression appliquée à la solution entre 10 10³Pa (100 mbar) et 3,5 10³ Pa (35 mbar) selon la même procédure que pour l'exemple A.

On récupère sur le filtre un produit cristallin, de couleur "blanc cassé", composé de cristaux arrondis de CL20 ε d'un diamètre médian de 236 µm, non maclés, de rapport d'allongement inférieur à 1,5 pour plus de 90 % d'entre eux (% en nombre), tels que montrés sur la figure 1B.

L'exemple D du tableau 4 concerne l'obtention de cristaux à chaud tout en contrôlant le paramètre de pression comme selon l'invention. Dans une solution proche de celle de l'exemple C (voir le tableau 4), on distille progressivement le solvant pendant 3h20 en portant la température de distillation à 67°C et en ajustant la pression appliquée à la solution légèrement en dessous du point d'ébullition du solvant, la pression variant alors entre 69,5 10³ Pa (695 mbar) et 35 10³ Pa (350 mbar). On récupère sur le filtre un produit cristallin, de couleur "blanc cassé", composé de cristaux sous la forme de bypiramide, le plus souvent maclés, de CL20 ε d'un diamètre médian de 213 µm, de rapport d'allongement supérieur à 2 pour plus de 90% d'entre eux (% en nombre). La figure 2B montre des cristaux, plus particulièrement des exemples de cristaux non maclés, obtenus par la procédé de l'exemple D. Comme pour l'exemple B, l'exemple D montre que l'ajustement du seul paramètre de pression (selon l'invention) ne permet pas d'obtenir la morphologie de cristaux désirée.

Les cristaux obtenus selon le procédé de l'invention présentent donc une forme arrondie favorable à l'obtention de matériaux énergétiques les incorporant à fort taux de charge.

De plus, ils présentent une sensibilité réduite aux tests de sensibilité pyrotechnique, par exemple, au test de transition déflagration détonation (TDD)*** et au test de mesure de vitesse de combustion à pression atmosphérique (épreuve de combustion linéaire du produit sous la forme pulvérulente disposé dans une gouttière) : voir les résultats indiqués dans le tableau 2 ci-après. Des précisions sur ledit test TDD sont données plus loin.

**Tableau 2**

| | Propriétés des cristaux obtenus | | |
|---|---|---|---|
| | Dmédian (µm) | TDD (phi 10 mm) | Vc gouttière (mm/s) |
| ***C (invention)*** | 236 | 150 mm combustion | 127 |
| | | 175 mm explosion | |
| ***D (exemple comparatif)*** | 213 | 75 mm combustion | 210 |
| | | 100 mm explosion | |

**Tableau 3**

| | Volume de la solution | % massique (solution) | | | Semence en cristaux de CL20 ε | | Conditions d'évaporation du solvant | | | Propriétés des cristaux obtenus | | | |
|---|---|---|---|---|---|---|---|---|---|---|---|---|---|
| ***Exemple*** | | CL20 | HFE7500 | Acétate d'éthyle | Dmédlan (µm) | Masse (kg) | T (°C) | Plage de vide (10³Pa) | Durée | Dmédian (µm) | ISI (J) | IAD (cartes) | Morpholog le des cristaux |
| *A* | 60L | 8,1 | 61,8 | 30,1 | 30 | 0,11 | 20 | 20 à 8 | 5h30 | 122 | 4 | 335 | Arrondie |
| ***B*** | 60L | 7,5 | 57,1 | 35,4 | 6 | 0,11 | 67 | 42,5 à 34,5 | 2h10 | 115 | 2,7 | > 360 | Bipyramid e, maclé |

**Tableau 4**

| | Volume de la solution | % massique (solution) | | | Semence en cristaux de CL20 ε | | Conditions d'évaporation du solvant | | | Propriétés des cristaux obtenus | | | |
|---|---|---|---|---|---|---|---|---|---|---|---|---|---|
| ***Exemple*** | | CL20 | Toluène | Acétone | Dmédian (µm) | Masse (kg) | T (°C) | Plage de vide (10³ Pa) | Durée | Dmédian (µm) | TDD phi 10 | Vc gouttière mm/s | Morphologie des cristaux |
| ***C*** | 60L | 9,3 | 72,6 | 18,1 | 150 | 0,1 | 20 | 10 à 3,5 | 5h00 | 236 | 150mm comb 175mm expl | 127 | Arrondie |
| *D* | 60L | 9,4 | 72,5 | 18,1 | 150 | 0,12 | 67 | 69,5 à 35 | 3h20 | 213 | 75mm comb 100mm expl | 210 | Bipyramide, maclé |

| | | | | | | | | | | | | | |
|---|---|---|---|---|---|---|---|---|---|---|---|---|---|
| ***ISI : L'épreuve réalisée correspond à celle décrite dans la norme NFT 70-500, elle-même semblable à l'épreuve ONU 3a)ii) issue des "Recommandations relatives au Transport des marchandises dangereuses - manuel d'épreuves et de critères, Quatrième édition révisée, ST/SG/AC.10/11/Rev.4, ISBN 92-1-239083-8ISSN 1014-7179*". Par une série minimale de 30 essais, on détermine l'énergie entraînant 50% (méthode de traitement des résultats de Bruceton) de résultats positifs d'une matière explosible soumise aux chocs d'un mouton. La matière à éprouver est confinée dans un dispositif en acier constitué de deux galets et d'une bague de guidage. En modifiant la masse et la hauteur de chute du mouton, on peut faire varier l'énergie de 1 à 50 J.* **IAD : L'épreuve est réalisée selon la norme NF T 70-502 (voir aussi ONU - Recommandations relatives au Transport des marchandises dangereuses - manuel d'épreuves et de critères, Quatrième édition révisée, ST/SG/AC.10/11/Rev.4, ISBN 92-1-239083-8ISSN 1014-7179 et STANAG 4488*). Elle consiste à déterminer la réactivité d'une substance explosive soumise à une détonation d'un relais d'amorçage au travers d'une barrière constituée de cartes d'acétate de cellulose. On détermine l'épaisseur limite de la barrière pour laquelle il n'y a pas d'amorçage de la détonation d'un second relais placé au contact de l'autre face de l'éprouvette.* *** *TDD : L'épreuve consiste à mesurer l'aptitude d'une masse de matériau divisé (lit de grains) à passer de la combustion à la détonation à la suite d'une inflammation, réalisée à la surface du lit, spécifiquement pour le CL20, sinon à la base du lit de poudre. L'épreuve N° 55 SNPE consiste à remplir un tube métallique de diamètre 40 mm (épreuve 55A) ou 10 mm (épreuve 558), de hauteur variable. Le tube est ouvert à une extrémité. La hauteur critique amenant à une réaction violente est déterminée à partir des effets remarqués sur le tube.* | | | | | | | | | | | | | |

## Revendications

1. Procédé d'obtention d'une charge de cristaux d'hexanitrohexaazaisowurtzitane, **caractérisé en ce qu'**il comprend :
- la préparation d'une solution saturée en hexanitrohexaazaisowurtzitane de forme polymorphe quelconque dans un mélange comprenant, d'une part, un solvant organique dudit hexanitrohexaazaisowurtzitane et, d'autre part, un non solvant organique dudit hexanitrohexaazaisowurtzitane ; ledit solvant étant plus volatil que ledit non solvant,
- l'ensemencement de cette solution saturée par quelques cristaux d'hexanitrohexaazaisowurtzitane, puis
- la concentration de la solution saturée ensemencée par évaporation au moins partielle du solvant ; ladite évaporation du solvant étant mise en oeuvre à une température comprise entre 35°C et 15°C et à une pression ajustée, à plus ou moins 1,2 10³ Pa (12 mbar), à la pression d'ébullition du solvant dans ladite solution, tout au long du processus d'évaporation dudit solvant de ladite solution.

2. Procédé selon la revendication 1, **caractérisé en ce que** ledit solvant organique est choisi dans le groupe constitué par les esters, les nitriles, les éthers, les cétones et leurs mélanges et/ou, avantageusement et, **en ce que** ledit non solvant organique est choisi dans le groupe constitué par les hydrocarbures aliphatiques, les hydrocarbures aliphatiques halogénés, les hydrocarbures aromatiques, les hydrofluoroéthers ininflammables et leurs mélanges.

3. Procédé selon la revendication 1 ou 2, **caractérisé en ce que** ladite évaporation du solvant est mise en oeuvre à une température comprise entre 35°C et 15°C et à une pression ajustée, à plus ou moins 5 10² Pa (5 mbar), à la pression d'ébullition du solvant dans ladite solution, tout au long du processus d'évaporation dudit solvant de ladite solution.

4. Procédé selon l'une quelconque des revendications 1 à 3, **caractérisé en ce qu'**au démarrage de l'évaporation, la pression est supérieure à la pression d'ébullition du solvant dans ladite solution.

5. Procédé selon l'une quelconque des revendications 1 à 4, **caractérisé en ce que** ledit solvant est évaporé pour l'obtention d'une suspension des cristaux dans un mélange solvant/non solvant ; ledit mélange solvant/non solvant renfermant moins de 15 %, avantageusement moins de 5%, en masse de solvant.

6. Procédé selon la revendication 5, **caractérisé en ce qu'**il comprend en outre le lavage, avec du non solvant, de ladite suspension.

7. Procédé selon l'une quelconque des revendications 1 à 6, **caractérisé en ce qu'**il comprend, en outre, la récupération des cristaux par filtration.

8. Procédé selon l'une quelconque des revendications 1 à 7, **caractérisé en ce que** ledit ensemencement est réalisé avec des cristaux de forme polymorphe ε ; la solution saturée renfermant avantageusement de l'hexanitrohexaazaisowurtzitane d'une forme polymorphe autre que la forme polymorphe ε.

9. Charge de cristaux susceptible d'être obtenue par le procédé selon l'une quelconque des revendications 1 à 8.

10. Charge de cristaux selon la revendication 9, **caractérisée en ce qu'**au moins 80 % des cristaux qu'elle referme présentent un rapport d'allongement inférieur ou égal à 1,5, avantageusement compris entre 1,5 et 1.

11. Matériau énergétique renfermant des cristaux d'hexanitrohexaazaisowurtzitane, **caractérisé en ce qu'**il renferme au moins une charge de cristaux, à l'état sec, selon la revendication 9 ou 10 et/ou au moins une charge de cristaux, à l'état sec, obtenue par le procédé selon l'une quelconque des revendications 1 à 8.

12. Procédé de fabrication d'un matériau énergétique selon la revendication 11, comprenant l'introduction des différents ingrédients constitutifs dudit matériau dans un malaxeur et leur malaxage au sein dudit malaxeur, **caractérisé en ce qu'**il comprend :
- l'introduction d'une suspension obtenue à la revendication 5 ou 6 dans ledit malaxeur, et
- l'extraction sous vide, totale ou quasi-totale, de la phase liquide de ladite suspension.

13. Procédé selon la revendication 12, **caractérisé en ce qu'**il comprend, en outre :
- la conservation, ou le transport, ou la conservation et le transport, ou le transport et la conservation, de ladite suspension, avant son introduction dans ledit malaxeur.

## Patentansprüche

1. Verfahren zum Erhalt einer Ladung von Hexanitrohexaazaisowurtzitan-Kristallen, **dadurch gekennzeichnet, dass** es umfasst:
- die Herstellung einer an Hexanitrohexaazaisowurtzitan von beliebiger polymorpher Form gesättigten Lösung in einer Mischung, die einerseits ein organisches Lösungsmittel des Hexanitrohexaazaisowurtzitan und andererseits ein organisches Nichtlösungsmittel des Hexanitrohexaazaisowurtzitan umfasst, wobei das Lösungsmittel flüchtiger als das Nichtlösungsmittel ist,
- das Beimpfen dieser gesättigten Lösung durch einige Hexanitrohexaazaisowurtzitan-Kristalle, anschließend
- die Konzentration der beimpften gesättigten Lösung durch wenigstens teilweises Verdampfen des Lösungsmittels; wobei das Verdampfen des Lösungsmittels bei einer Temperatur im Bereich zwischen 35 °C und 15 °C und bei einem Druck, der, bis auf mehr oder weniger 1,2 10³ Pa (12 mbar), auf den Siededruck des Lösungsmittels in der Lösung, während des gesamten Verdampfungsprozesses des Lösungsmittels der Lösung eingestellt ist, durchgeführt wird.

2. Verfahren nach Anspruch 1, **dadurch gekennzeichnet, dass** das organische Lösungsmittel ausgewählt ist aus der Gruppe bestehend aus den Estern, den Nitrilen, den Ethern, den Ketonen und ihren Mischungen und/oder, vorteilhafterweise und, dass das organische Nichtlösungsmittel ausgewählt ist aus der Gruppe bestehend aus den aliphatischen Kohlenwasserstoffen, den halogenierten aliphatischen Kohlenwasserstoffen, den aromatischen Kohlenwasserstoffen, den unentzündlichen Hydrofluorethern und ihren Mischungen.

3. Verfahren nach Anspruch 1 oder 2, **dadurch gekennzeichnet, dass** das Verdampfen des Lösungsmittels bei einer Temperatur im Bereich zwischen 35 °C und 15 °C und bei einem Druck, der, bis auf mehr oder weniger 5 10² Pa (5 mbar), auf den Siededruck des Lösungsmittels in der Lösung, während des gesamten Verdampfungsprozesses des Lösungsmittels der Lösung eingestellt ist, durchgeführt wird.

4. Verfahren nach einem der Ansprüche 1 bis 3, **dadurch gekennzeichnet, dass** beim Starten der Verdampfung der Druck höher als der Siededruck des Lösungsmittels in der Lösung ist.

5. Verfahren nach einem der Ansprüche 1 bis 4, **dadurch gekennzeichnet, dass** das Lösungsmittel für den Erhalt einer Suspension der Kristalle in einer Lösungsmittel/Nichtlösungsmittel-Mischung verdampft wird; wobei die Lösungsmittel/Nichtlösungsmittel-Mischung weniger als 15, vorteilhafterweise weniger als 5 Massenprozent Lösungsmittel enthält.

6. Verfahren nach Anspruch 5, **dadurch gekennzeichnet, dass** es ferner das Waschen, mit Nichtlösungsmittel, der Suspension umfasst.

7. Verfahren nach einem der Ansprüche 1 bis 6 **dadurch gekennzeichnet, dass** es ferner die Gewinnung der Kristalle durch Filtration umfasst.

8. Verfahren nach einem der Ansprüche 1 bis 7, **dadurch gekennzeichnet, dass** das Beimpfen mit Kristallen von ε polymorpher Form vollzogen wird; wobei die gesättigte Lösung vorteilhafterweise Hexanitrohexaazaisowurtzitan von einer anderen polymorphen Form als der ε polymorphen Form enthält.

9. Kristallladung, die geeignet ist, durch das Verfahren nach einem der Ansprüche 1 bis 8 erhalten zu werden.

10. Kristallladung nach Anspruch 9, **dadurch gekennzeichnet, dass** wenigstens 80 % der Kristalle, die sie enthält, ein Streckungsverhältnis kleiner oder gleich 1,5, vorteilhafterweise zwischen 1,5 und 1 aufweisen.

11. Energetisches Material, das Hexanitrohexaazaisowurtzitan-Kristalle enthält, **dadurch gekennzeichnet, dass** es wenigstens eine Kristallladung, in trockenem Zustand, nach Anspruch 9 oder 10 und/oder wenigstens eine Kristallladung, in trockenem Zustand, erhalten durch das Verfahren nach einem der Ansprüche 1 bis 8 enthält.

12. Verfahren zur Herstellung eines energetischen Materials nach Anspruch 11, umfassend das Einbringen der verschiedenen Bestandteile des Materials in einen Mischer und deren Mischen in dem Mischer, **dadurch gekennzeichnet, dass** es umfasst:
- das Einbringen einer bei Anspruch 5 oder 6 erhaltenen Suspension in den Mischer und
- das vollständige oder quasi vollständige Vakuumextrahieren der flüssigen Phase der Suspension.

13. Verfahren nach Anspruch 12, **dadurch gekennzeichnet, dass** es ferner umfasst:
- die Aufbewahrung oder den Transport oder die Aufbewahrung und den Transport oder den Transport und die Aufbewahrung der Suspension vor ihrem Einbringen in den Mischer.

## Claims

1. A process for obtaining a charge of hexanitrohexaazaisowurtzitane crystals, **characterized in that** it comprises:
- preparing a saturated solution of any polymorphic form of hexanitrohexaazaisowurtzitane in a mixture comprising, on the one hand, an organic solvent for said hexanitrohexaazaisowurtzitane and, on the hand, an organic nonsolvent for said hexanitrohexaazaisowurtzitane, said solvent being more volatile than said nonsolvent;
- seeding this saturated solution with a few hexanitrohexaazaisowurtzitane crystals; and then
- concentrating the seeded, saturated solution by at least partial evaporation of the solvent, said evaporation of the solvent being performed at a temperature of between 35°C and 15°C and at a pressure adjusted, to plus or minus 1.2 x 10³ Pa (12 mbar), to the boiling pressure of the solvent in said solution, throughout the process of evaporating said solvent from said solution.

2. The process according to claim 1, **characterized in that** said organic solvent is selected from the group consisting of esters, nitriles, ethers, ketones, and mixtures thereof, and/or, advantageously and, **in that** said organic nonsolvent is selected from the group consisting of aliphatic hydrocarbons, halogenated aliphatic hydrocarbons, aromatic hydrocarbons, nonflammable hydrofluoroethers, and mixtures thereof.

3. The process according to claim 1 or 2, **characterized in that** said evaporation of the solvent is performed at a temperature of between 35°C and 15°C and at a pressure adjusted, to plus or minus 5 x 10² Pa (5 mbar), to the boiling pressure of the solvent in said solution, throughout the process of evaporating said solvent from said solution.

4. The process according to any of claims 1 to 3, **characterized in that** the pressure on startup of the evaporation is greater than the boiling pressure of the solvent in said solution.

5. The process according to any of claims 1 to 4, **characterized in that** said solvent is evaporated to give a suspension of the crystals in a solvent/nonsolvent mixture, said solvent/nonsolvent mixture containing less than 15%, advantageously less than 5%, by mass of solvent.

6. The process according to claim 5, **characterized in that** it further comprises washing said suspension with the nonsolvent.

7. The process according to any of claims 1 to 6, **characterized in that** it further comprises recovering the crystals by filtration.

8. The process according to any of claims 1 to 7, **characterized in that** said seeding is carried out with crystals of polymorphic form ε, the saturated solution advantageously containing hexanitrohexaazaisowurtzitane in a polymorphic form other than the ε polymorphic form.

9. A charge of crystals which is obtainable by the process according to any of claims 1 to 8.

10. The charge of crystals according to claim 9, **characterized in that** at least 80% of the crystals in the charge have an elongation ratio of less than or equal to 1.5, advantageously of between 1.5 and 1.

11. An energetic material comprising hexanitrohexaazaisowurtzitane crystals, **characterized in that** it comprises at least one charge of crystals, in the dry state, according to claim 9 or 10 and/or at least one charge of crystals, in the dry state, obtained by the process as claimed according to any of claims 1 to 8.

12. A process for manufacturing an energetic material according to claim 11, comprising introducing the various constituent ingredients of said material into a mixer and mixing them within said mixer, said process being **characterized in that** it comprises:
- introducing a suspension obtained in claim 5 or 6 into said mixer; and
- extracting all or virtually all of the liquid phase from said suspension under vacuum.

13. The process according to claim 12, **characterized in that** it further comprises:
- storing, or transporting, or storing and transporting, or transporting and storing said suspension before it is introduced into said mixer.
